(19) **Europäisches Patentamt / European Patent Office / Office européen des brevets**

(11) **EP 2 294 044 B1**

(12) **EUROPEAN PATENT SPECIFICATION**

(45) Date of publication and mention of the grant of the patent:
**13.07.2016 Bulletin 2016/28**

(21) Application number: **08763845.8**

(22) Date of filing: **28.04.2008**

(51) Int Cl.:
**C07C 41/16** (2006.01)     **C07C 43/12** (2006.01)

(86) International application number:
**PCT/IT2008/000293**

(87) International publication number:
**WO 2009/133575 (05.11.2009 Gazette 2009/45)**

(54) **FLUOROALKYLOXY ALKANES, PROCESS FOR PRODUCTION AND USES THEREOF**

FLUORALKYLOXYALKANE, VERFAHREN ZUR HERSTELLUNG UND ANWENDUNGEN DAVON

FLUOROALKYLOXY-ALCANES, PROCÉDÉ DE PRODUCTION ET UTILISATIONS

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**16.03.2011 Bulletin 2011/11**

(73) Proprietor: **AL.CHI.MI.A. S.r.l.**
**35020 Ponte S. Nicolò (Padova) (IT)**

(72) Inventors:
• **CONTE, Lino**
  **I-35028 Piove Di Sacco (Padova) (IT)**
• **ZAGGIA, Alessandro**
  **I-35100 Padova (IT)**
• **BECCARO, Mauro**
  **I-35010 Cadoneghe (Padova) (IT)**
• **BETTINI, Enrico**
  **I-30032 Fiesso D'Artico (Venezia) (IT)**
• **SIGNORI, Paolo**
  **I-37131 Verona (IT)**

(74) Representative: **Ponchiroli, Simone**
**Ruffini Ponchiroli e Associati S.r.l.**
**Via Caprera, 6**
**37126 Verona (IT)**

(56) References cited:
**WO-A-02/03958       WO-A-96/40834**

WO-A-97/39081       WO-A-2005/117850
JP-A- 10 175 899     US-A- 6 060 626

• C. H. DEPUY, S. GRONERT, A. MULLIN, V. M. BIERBAUM: "Gas-phase SN2 and E2 reactions of alkyl halides" J. AM. CHEM. SOC., vol. 112, 1990, page 8650-8655, XP002517121
• SELVE CLAUDE ET AL: "Synthesis of monodisperse perfluoroalkyl oxyethylene surfactants with methoxy capping: surfactants of high chemical inertness" JOURNAL OF THE CHEMICAL SOCIETY, CHEMICAL COMMUNICATIONS, CHEMICAL SOCIETY. LETCHWORTH, GB, 1 January 1990 (1990-01-01), pages 911-912, XP009112323 ISSN: 0022-4936
• M. B. SMITH, J. MARCH: "March's Advanced Organic Chemistry, Reactions, Mechanisms, and Structure, 5th Edition" 2001, WILEY-INTERSCIENCE , NEW YORK, USA, ISBN 0-471-58589-0 , XP002517122 chapter 10-12 page 477
• CHU ET AL: "New fluorous/organic biphasic systems achieved by solvent tuning" TETRAHEDRON, ELSEVIER SCIENCE PUBLISHERS, AMSTERDAM, NL, vol. 63, no. 39, 16 August 2007 (2007-08-16), pages 9890-9895, XP022201081 ISSN: 0040-4020
• BAZHIN D N ET AL: "Synthesis of Polyfluorinated Ethers", RUSSIAN JOURNAL OF APPLIED CHEMISTRY, NAUKA/INTERPERIODICA, MO, vol. 78, no. 10, 1 October 2005 (2005-10-01), pages 1646-1650, XP019299429, ISSN: 1608-3296

**Description**

The present invention relates to fluoroalkyloxy alkanes, to their synthesis process and to the use thereof for medical applications, in particular as liquids in ophthalmic treatments.

It is known about the use of liquid perfluorocarbons in ophthalmology, e.g. as tamponade liquids in operations for the treatment of retinal detachment or as vitreous body substitutes (see for instance patent application EP 0 089 232 A2).

Thanks to their high density (generally above 1.6 g/cm$^3$) and to their extremely low surface tension (below 25 mN/m), liquid perfluorocarbons allow a complete unfolding and a correct re-positioning of the retina so as to favor the re-adhesion thereof. Perfluorocarbons are temporarily chemically inert and physiologically biocompatible products, if completely fluorinated. As a matter of fact, the presence, if any, of - $CHF$-$CF_2$- groups can give rise to dehydrofluorination with HF formation, resulting in the subsequent reduction of biocompatibility. Moreover, after their action as tamponade liquids during surgery is ended, perfluorocarbons cannot be kept in contact with the retina for long, since the high density can give rise, in time, to phenomena of thinning of the lower retina. Therefore, the use of perfluorocarbons is recommended only intra-surgically. If left in the eye also after surgery, these should anyhow be removed and replaced with a permanent tamponade liquid, such as a silicone oil.

It is known about the use of silicone oils (in particular polydimethylsiloxane) as tamponade liquids during ophthalmic surgery. These products, however, have some limitations related in particular to the fact that silicone oils have too low a density, usually of about 0.97 g/cm$^3$ and thus lower than water density, and cannot ensure a sufficient pressure in the retina, especially the lower retina, since they tend to come to the surface of the aqueous medium constituting the vitreous body.

Recently, other fluorinated compounds have been proposed as liquids that can be used in ophthalmic applications.

For instance, patent US 6,262,126 B1 describes partially fluorinated alkanes with formula $R_F R_H$ or $R_F R_H R_F$, wherein $R_F$ is a linear or branched perfluoroalkyl group and $R_H$ is a linear or branched, saturated alkyl group. The number of carbon atoms in the perfluorinated moiety can vary from 1 to 20, whereas for the alkyl moiety the number of carbon atoms is usually from 3 to 20. These products are obtained by reaction of perfluoroalkyl iodides with alkenes or $\alpha,\omega$-dienes, with HI elimination, and subsequent hydrogenation in the presence of a platinum catalyst or by reaction with tributyltin hydride. These products have a density generally of from 1.1 to 1.7 g/cm$^3$, thus lower than the density of perfluoroalkanes. Since they have a certain solubility in non-fluorinated compounds, they can be used as tamponade liquids in mixture with silicone oils, with the goal of obtaining a density slightly higher than water.

International patent application WO 2005/117850 describes partially fluorinated ethers and the use thereof as tamponade agents in mixture with a liquid silicone or a liquid perfluorocarbon. These partially fluorinated ethers have a specific gravity of from 1.1 to 1.5 and the following formula:

$$Rf\ (CH_2)_n O(CH_2)_n Rf$$

wherein Rf is a fluorinated, monovalent, saturated organic C1-C4 group and n is 3 or 4. Decafluoro-di-n-pentyl ether (DFPE) is particularly preferred. Preferably, the mixture comprises from 10 to 20% of DFPE and from 80 to 90% of silicone oil.

US patent 6,060,626 discloses a fluorine-containing compound having functions corresponding to those of fluorine, which is said to stable under various conditions, excellent in compatibility with other solvents, and usable as solvents, cosmetics, detergents, emulsifiers, surface finishing agents, lubricants, as well as lubricants or oils in the field of semiconductor/electronics. Such a fluorine-containing ether compounds is represented by the general formula

$$R_f - (CH_2)_n - O - R_1$$

wherein, Rf represents a straight or branched C1-20 perfluoroalkyl or fluoroalkyl group, R1 represents a straight or branched C3-9 alkyl or a C3-9 cycloalkyl group, and n is a number from 1 to 8.

Said compounds are obtained by reacting a fluorine containing hydroxy compound with a carbonyl compound in an atmosphere of hydrogen or in hydrogen gas. A similar process is disclosed also in JP 10 175899.

International application EP 02/03958 then discloses a pharmaceutical aerosol formulation intended for inhalation which contains an active substance, an aerosol propellant, a polar flurorinated molecule and an excipient.

C.H. Depuy et al: "Gas-phase SN2 and E2 reactions of alkyl halides", J. Am. Chem. Soc., vol. 112, 1990, pages 8650-8655, discloses the gas-phase synthesis of $C_2F_5CH_2OR$ (R being Me, Et, Pr, [i]Pr, [i]Bu) starting from $C_2F_5CH_2OH$.

Selve Calude et al: "Synthesis of monodisperse perfluoroalkyl oxyethylene surfactants with methoxy capping: surfactants of high chemical inertness", Journal of the Chemical Society, Letchworth, GB, 1 January 1990, pages 911-912, discloses the preparation of some linear molecules having the general structure

$$Y(CF_2)_nCH_2O(C_2H_4O)_pZ$$

(Y being F or H and Z being H or Me) using Williamson condensation with solid KOH, solvent and phase transfer reagent.

M.B. Smith, J. March: "March's advanced Organic Chemistry, Reactions, Mechanisms, and Structure, 5th Edition", 2001, Wiley-Interscience New York, USA, discloses a general description of the Williamson Reaction. According to it the normal method for carrying it out involves treatment of the halide with alkoxide or aroxide ion prepared from an alcohol or phenol, but is also possible to mix the halide and alcohol or phenol directly with $CS_2CO_3$ in acetonitrile or with solid KOH in $Me_2SO$.

In Chu et al: "New fluorous/organic biphasic systems achieved by solvent tuning", Tetrahedron, Elsevier Science Publishers, Amsterdam. NL, vol. 63, no. 39, 16 August 2007, pages 9890-9895, miscibility tests between sixty pairs of fluorous and organic solvents have been performed, and a number of biphasic systems based on hydrofluoroether solvents have been identified.

WO 96/40834 discloses compositions comprising a hydrofluoroether or fluoroether of the formula $C_aF_bH_{2a+2-b}O_c$ wherein a = 3 to 6, b = 1 to 14 and c = 1 or 2; an acyclic hydrofluorocarbon or fluorocarbon of the formula $C_dF_eH_{2d+2-e}$ wherein d = 4 to 6 and e = 1 to 14; a cyclic hydrofluorocarbon or fluorocarbon of the formula $C_mF_nH_{2m-n}$ wherein m = 4 to 6 and n = 1 to 12; a fluoroalcohol of the formula $C_rF_sH_{2r+1-s}OH$ wherein r = 4 to 6 and s = 1 to 13; or perfluoro-n-methylmorpholine. Said compositions are said to useful as refrigerants, cleaning agents, aerosol propellants, heat transfer media, gaseous dielectrics, fire extinguishing agents, expansion agents for polymers such as polyolefins and polyurethanes, and as power cycle working fluids.

WO 97/39081 discloses compositions of 1,1,2,2,3,3,4,4-octafluorobutane and a compound of the formula selected from the group consisting of a 4-6 carbon linear or a cyclic hydrofluorocarbon or fluorocarbon, a 3-5 carbon fluoroether, a fluorinated alcohol, perfluoro-n-methylmorpholine, and an ether are disclosed. These compositions, which may be azeotropic or azeotrope-like, are said to be usable as refrigerants, in small turbine high speed compressors. They are said also to be useable as refrigerants, cleaning agents, expansion agents for polyolefins and polyurethanes, aerosol propellants, heat transfer media, gaseous dielectrics, fire extinguishing agents, power cycle working fluids, polymerization media, particulate removal fluids, carrier fluids, buffing abrasive agents, displacement drying agents or as an anesthetic.

Finally, Bazhin D N et al: "synthesis of polyfluorinated Ethers", Russian Journal of applied Chemistry, nauka/Interperiodica, MO, vol. 78, n. 10, 1 October 2005, pages 1646-1650, describes different processes for the production of compounds defined by the formula

$$H(CF_2CF_2)_nCH_2OR.$$

According to it, for the production of such compounds classical Williamson reaction is not appropriate, while best results can be obtained by means of a process comprising two subsequent steps. In the first step, tosyl derivatives of alcohols are prepared; in the second step these tosyl derivatives are reacted with telomeric alcohols under the conditions of phase-transfer catalysis.

The Applicant has faced the problem of finding liquids with high biocompatibility, high purity degree and suitable density values so that they can be used as liquids in ophthalmic treatments, in particular as tamponade liquids during surgical operations on the retina, which are able to exert a sufficient pressure during said operation so as to enable a correct re-positioning and a sufficient adhesion of the retina to the adjacent eye components, and which can be retained at the same time inside the eye structure for an undetermined lapse of time without damaging the retina itself.

The Applicant has found that this aim and others of the invention can be achieved by way of fluoroalkyloxy alkanes as defined hereinbelow, which both meet the above requirements and have a high solubility in unfluorinated liquids, in particular silicone oils, so as to enable the preparation of mixtures in a wide range of concentrations, which are stable at working temperatures, as better described in the following, without showing phase separation.

According to a first aspect, the present invention therefore relates to fluoroalkyloxy alkanes as defined in claim 1, having formula (I):

$$R_F-R_1-O-R_2 \qquad (I)$$

wherein:

$R_F$ is a linear perfluoroalkyl group, having 4 or 6 carbon atoms;
$R_1$ is a linear non-fluorinated alkylene group, having 2, carbon atoms;
$R_2$ is a linear unfluorinated alkyl group, having 3 carbon atoms.

Preferably, fluoroalkyloxy alkanes having formula (I) have a density which may vary from 1.2 to 1.5 g/cm$^3$, measured at 20°C.

According a second aspect, the present invention relates to a process for preparing a fluoroalkyloxy alkane having formula (I), which comprises reacting a fluorinated alcohol having formula (II):

$$R_F\text{-}R_1\text{-}OH \qquad (II)$$

wherein $R_F$ and $R_1$ are as defined above, with an alkyl halide having formula (III):

$$R_2\text{-}X \qquad (III) :$$

wherein $R_2$ is as defined above and X is a halogen selected from Br and I, preferably Br.

Preferably, the reaction is carried out at a basic pH, for instance by adding an aqueous solution of a strong base, such as NaOH or KOH, or a tertiary amine, e.g. triethylamine, in a solvent comprising water and at.least one at least partially water-soluble organic solvent, which is able to solubilize at least partially the fluorinated alcohol having formula (II). Example of suitable organic solvents are: acetone, N,N-dimethyl formamide, N-methyl pyrrolidone, tetrahydrofurane.

In order to promote the reaction between the fluorinated alcohol having formula (II), which is at least partially soluble in the aqueous medium, and the alkyl halide having formula (III), which is basically insoluble in the aqueous medium, the reaction mixture is added with at least one phase transfer catalyst, e.g. a quaternary ammonium salt, in particular a tetraalkyl ammonium salt or a benzyltrialkyl ammonium salt, e.g. benzyltriethyl ammonium chloride.

The reaction is preferably carried out at a temperature of from 25°C to 100°C, preferably from 40°C to 70°C, for a period of time generally of from 2 to 40 hours, preferably from 4 to 10 hours.

At the end of the reaction, after separating the aqueous phase, the organic phase can be purified, e.g. by distillation..In alternative or in addition to distillation, the end product can be purified by passage in a chromatographic column containing, for instance, silica or alumina.

According to a further aspect, the present invention relates to a mixture of at least one fluoroalkyloxy alkane having formula (I) and at least one silicone oil.

Preferably, the aforesaid mixture comprises from 10% to 95% by weight, more preferably from 20% to 80% by weight, of at least one fluoroalkyloxy alkane having formula (I), and from 5% to 90% by weight, more preferably from 20 to 80% by weight, of at least one silicone oil.

Preferably, said at least one silicone oil has a viscosity of from 100 to 100,000 cS (centistokes), measured at 20°C. Preferably, said silicone oil is a polydimethylsiloxane.

According to a further aspect, the present invention relates to a fluoroalkyloxy alkane having formula (I) for use as a medicament, in particular in ophthalmology.

According a further aspect, the present invention relates to a fluoroalkyloxy alkane having formula (I) for use as a tamponade liquid in an operation for the treatment of retinal detachment.

According a further aspect, the present invention relates to a fluoroalkyloxy alkane having formula (I) for use as a vitreous body substitute. This use can be either permanent or temporary, usually for at least 12 months.

According a further aspect, the present invention relates to a fluoroalkyloxy alkane having formula (I) for use as an agent for oxygenating biological tissues.

According to a further aspect, the present invention relates to a fluoroalkyloxy alkane having formula (I) as a drug carrier.

According to a further aspect, the present invention relates to a mixture of at least one fluoroalkyloxy alkane having formula (I) and at least one silicone oil for use as a medicament in.ophthalmology.

According to a further aspect, the present invention relates to a mixture of at least one fluoroalkyloxy alkane having formula (I) and at least one silicone oil for use as a tamponade liquid in an operation for the treatment of retinal detachment.

According to a further aspect, the present invention relates to a mixture of at least one fluoroalkyloxy alkane having formula (I) and at least one silicone oil for use as a vitreous body substitute.

According to a further aspect, the present invention relates to the use of a fluoroalkyloxy alkane having formula (I) for producing a medicament for use in ophthalmology.

According to a further aspect, the present invention relates to the use of a fluoroalkyloxy alkane having formula (I) for producing a tamponade liquid for use in an operation for the treatment of retinal detachment.

According to a further aspect, the present invention relates to the use of a fluoroalkyloxy alkane having formula (I) for producing a vitreous body substitute.

According to a further aspect, the present invention relates to the use of a mixture of at least one fluoroalkyloxy alkane having formula (I) and at least one silicone oil for producing a medicament for use in ophthalmology.

According to a further aspect, the present invention relates to the use of a mixture of at least one fluoroalkyloxy alkane having formula (I) and at least one silicone oil for producing a tamponade liquid for use in an operation for the treatment of retinal detachment.

According to a further aspect, the present invention relates to the use of a mixture of at least one fluoroalkyloxy alkane having formula (I) and at least one silicone oil for producing a vitreous body substitute.

The present invention will now be disclosed in greater detail by way of some working examples, which are provided as mere non-limiting examples.

EXAMPLE 1

To a mixture, kept under stirring, of $C_6F_{13}CH_2CH_2OH$ (252 g, 0.687 moles), tetrahydrofurane (350 ml) and cyclohexane (300 ml) at 25°C, an aqueous solution of NaOH (400 ml, 50%) was added drop-wise. After stirring for 2 hours, benzyltriethyl ammonium chloride (25 g, 0.11 moles) as phase transfer catalyst, and then 1-bromopropane (170 g, 1.38 moles) were added. The reaction mixture was kept under stirring at 40°C for 40 hours, then at 70°C for 8 hours. The resulting mixture was poured into water and the organic phase thus obtained was washed two times with water. After removing the fraction having a low boiling point, the raw product was treated with toluene 2,4-diisocyanate, in order to remove unreacted fluorinated alcohol, and then subjected to distillation at reduced pressure, thus obtaining 200 g of $C_6F_{13}CH_2CH_2O(CH_2)_2CH_3$ (yield 71%, boiling point 174°C, $n_D^{20}$ 1.3245). Spectrographic data confirm the obtained structure: GC/MS m/z at 405 (M-H)[+], 377 (M-CH$_2$CH$_3$)[+], 73 (-CH$_2$OCH$_2$CH$_2$CH$_3$)[+], 43 (-CH$_2$CH$_2$CH$_3$)[+]; for NMR data see Tables 1-2.

EXAMPLE 2

To a mixture, kept under stirring, of $C_4F_9CH_2CH_2OH$ (200 g, 0.757 moles), tetrahydrofurane (350 ml) and cyclohexane (300 ml) at 25°C, an aqueous solution of NaOH (400 ml, 50%) was added drop-wise. After stirring for 2 hours, benzyltriethyl ammonium chloride (25 g, 0.11 moles) as phase transfer catalyst, and then 1-bromopropane (170 g, 1.38 moles) were added. The reaction mixture was kept under stirring at 40°C for 40 hours, then at 70°C for 8 hours. The resulting mixture was poured into water and the organic phase thus obtained was washed two times with water. After removing the fraction having a low boiling point, the raw product was subjected to distillation at reduced pressure, thus obtaining 200 g of $C_4F_9CH_2CH_2O(CH_2)_2CH_3$ (yield 86%, boiling temp. 147°C, $n_D^{20}$ 1.3255). Spectrographic data confirm the obtained structure: GC/MS m/z at 305 (M-H)[+], 377 (M-CH$_2$CH$_3$)[+], 73 (-CH$_2$OCH$_2$CH$_2$CH$_3$)[+], 43 (-CH$_2$CH$_2$CH$_3$)[+]; for NMR data see Tables 1-2.

EXAMPLE 3

The same fluoroalkyloxy alkanes as in Examples 1-2 were prepared following an alternative procedure. A mixture comprising 0.1 moles of fluorinated alcohol, 60 ml of N-methyl-2-pyrrolidone, 0.2 moles of 1-bromoalkane, and 60 ml of a 45% aqueous solution of KOH was heated under stirring for 5 hours at 50°C, then at 70°C for 2 hours so as to complete the reaction. The raw reaction mixture was filtered so as to remove the formed KBr, and then diluted in 20 ml of water; the organic phase thus obtained comprised the desired fluoroalkyloxy alkane (yield: 80%).

As far as NMR data are concerned, these were obtained in acetone-d6. Indexes identifying the various carbon atoms are assigned as disclosed above for the product $CF_3(CF_2)_3CH_2CH_2O(CH_2)_2CH_3$:

$$CF_3(a)-CF_2(b)-CF_2(c)-CF_2(d)-CH_2(y)-CH_2(x)-O-CH_2(\gamma)-CH_2(\beta)-CH_3(\alpha)$$

TABLE 1

| ($^{19}$F NMR DATA) | | | | | | |
|---|---|---|---|---|---|---|
| Product | CF$_3$(a) | CF$_2$(b) | CF$_2$(c) | CF$_2$(d) | CF$_2$(e) | CF$_2$(f) |
| $CF_3(CF_2)_3CH_2CH_2O(CH_2)_2CH_3$ | -84.2 t (3F) | -128.9 m (2F) | -127.3 m (2F) | -116.2 m (2F) | - | - |
| $CF_3(CF_2)_5CH_2CH_2O(CH_2)_2CH_3$ | -83.9 t (3F) | -129.1 m (2F) | -126.3 m (2F) | -125.6 m (2F) | -124.6 m (2F) | -116.0 m (2F) |

TABLE 2

| ($^1$H NMR DATA) | | | | | | | |
|---|---|---|---|---|---|---|---|
| Product | $CH_3(\alpha)$ | $CH_2(\beta)$ | $CH_2(\gamma)$ | $CH_2(\delta)$ | $CH_2(\epsilon)$ | $CH_2(x)$ | $CH_2(y)$ |
| $CF_3(CF_2)_3CH_2CH_2O(CH_2)_2CH_3$ | 0.90 t(3H) | 1.56 tq(2H) | 3.41 t (2H) | - | - | 3.73 t(2H) | 2.49 ttt(2H) |
| $CF_3(CF_2)_5CH_2CH_2O(CF_2)_2CH_3$ | 0.90 t (3H) | 1.55 tq(2H) | 3.42 t (2H) | - | - | 3.74 t (2H) | 2.50 ttt (2H) |

EXAMPLE 4

Various mixtures comprising a fluoroalkyloxy alkane obtained according to Example 2 and a silicone oil having a viscosity of 1,000 cS (at 20°C) were prepared.

Table 3 contains the amounts of fluoroalkyloxy alkane in the various mixtures (expressed as weight % on the total weight of the mixture), and the result in terms of clarity of the solution at different temperatures.

TABLE 3

| Product | 20% | 33% | 43% | 50% | 60% | 90% |
|---|---|---|---|---|---|---|
| $CF_3(CF_2)_3CH_2CH_2O(CH)_2CH_3$ | n.d. | ++ | ++ | ++ | ++ | ++ |
| ++: the mixture is clear after stirring at room temperature | | | | | | |

EXAMPLE 5

Various mixtures comprising a fluoroalkyloxy al-kane obtained according to Example 2 and a silicone oil having a viscosity of 5,000 cS (at 20°C) were prepared.

Table 4 contains the amounts of fluoroalkyloxy alkane in the various mixtures (expressed as weight % on the total weight of the mixture), and the result in terms of clarity of the solution at different temperatures.

TABLE 4

| Product | 20% | 33% | 43% | 50% | 60% | 90% |
|---|---|---|---|---|---|---|
| $CF_3(CF_2)_3CH_2CH_2O(CH_2)_2CH_3$ | n.d. | ++ | ++ | ++ | ++ | ++ |
| ++: the mixture is clear after stirring at room temperature | | | | | | |

EXAMPLE 6

The following mixtures (weight %) comprising the fluoroalkyloxy alkanes obtained according to Example 2 with silicone oils having different viscosity values were prepared:

TABLE 5

| | Mixture 1 | Mixture 2 |
|---|---|---|
| $CF_3(CF_2)_3CH_2CH_2O(CH_2)_2CH_3$ | 90 | 90 |
| Silicone oil 1,000 cS (20°C) | 10 | -- |
| Silicone oil 5,000 cS (20°C) | - | 10 |

The aforesaid mixtures were cooled at 4°C. After cooling, Mixture 1 remained perfectly clear, whereas Mixture 2 showed a phase separation; after stirring opalescence was observed; after being brought to room temperature, Mixture 2 became clear after stirring.

These tests show the high solubility of fluoroalkyloxy alkanes according to the present invention with silicone oils in a wide range of concentrations, temperature and viscosity of said oils.

**Claims**

1. Fluoroalkyloxy alkane having the formula:

$$CF_3(CF_2)_5CH_2CH_2O(CH_2)_2CH_3$$

or

$$CF_3(CF_2)_3CH_2CH_2O(CH_2)_2CH_3.$$

2. A process for preparing a fluoroalkyloxy alkane of claim 1, the fluoroalkyloxy alkane having formula (I):

$$R_F\text{-}R_1\text{-}O\text{-}R_2 \qquad (I)$$

wherein:

   $R_F$ is a linear perfluoroalkyl group, having 4 or 6 carbon atoms;
   $R_1$ is a linear, non-fluorinated alkylene group, having 2 carbon atoms;
   $R_2$ is a linear, non-fluorinated alkyl group, having 3 carbon atoms;

   comprising reacting a fluorinated alcohol having formula (II):

$$R_F\text{-}R_1\text{-}OH \qquad (II)$$

   wherein $R_F$ and $R_1$ are as defined above, with an alkyl halide having formula (III):

$$R_2\text{-}X \qquad (III)$$

   wherein $R_2$ is as defined above and X is a halogen selected from Br and I, preferably Br,
   wherein the reaction is carried out at a basic pH in a solvent comprising water and at least one at least partially water-soluble organic solvent, which is able to solubilize at least partially the fluorinated alcohol having formula (II).

3. The process according to claim 2, further comprising adding to the reaction mixture at least one phase transfer catalyst.

4. The process according to claim 2 or 3, wherein the reaction is carried out at a temperature of 25°C to 100°C, preferably of 40°C to 70°C, for a period of time generally of from 2 to 40 hours, preferably from 4 to 10 hours.

5. A mixture of at least one fluoroalkyloxy alkane according to claim 1 and at least one silicone oil.

6. The mixture according to claim 5, comprising from 10% to 95% by weight, preferably from 20% to 80% by weight, of at least one fluoroalkyloxy alkane having formula (I), and from 5% to 90% by weight, preferably from 20% to 80% by weight, of at least one silicone oil.

7. The mixture according to claim 5 or 6, wherein said at least one silicone oil has a viscosity of from 100 to 100.000 cS (centistokes), measured at 20°C.

8. A fluoroalkyloxy alkane according to claim 1 for use as a medicament, in particular in ophthalmology.

9. The fluoroalkyloxy alkane according to claim 1 for use as a tamponade liquid in an operation for the treatment of retinal detachment.

10. The fluoroalkyloxy alkane according to claim 1 for use as a vitreous body substitute.

11. The fluoroalkyloxy alkane according to claim 1 for use as an agent for oxygenating biological tissues.

12. The fluoroalkyloxy alkane according to claim 1 for use as a drug carrier.

**13.** The mixture according to any of the claims from 5 to 7 for use as a medicament in ophthalmology.

**14.** The mixture according to any of the claims from 5 to 7 for use as a tamponade liquid in an operation for the treatment of retinal detachment.

**15.** The mixture according to any of the claims from 5 to 7 for use as a vitreous body substitute.

**Patentansprüche**

**1.** Fluoralkyloxalkan, enthaltend die folgende Formel:

$$CF_3(CF_2)_5CH_2CH_2O(CH_2)_2CH_3$$

oder

$$CF_3(CF_2)_3CH_2CH_2O(CH_2)_2CH_3$$

**2.** Verfahren zur Herstellung von Fluoralkyloxalkan nach Patentanspruch 1, wobei das Fluoralkyloxalkan folgende Formel (I) hat:

$$R_F\text{-}R_1\text{-}O\text{-}R_2 \qquad (I)$$

wobei:

$R_F$ eine lineare Perfluoralkylgruppe ist, die 4 oder 6 Kohlenstoffatome hat;
$R_1$ eine lineare, nicht-fluorierte Alkylengruppe ist, die 2 Kohlenstoffatome hat;
$R_2$ eine lineare, nicht-fluorierte Alkylgruppe ist, die 3 Kohlenstoffatome hat;

enthaltend reagierend einen fluorierten Alkohol mit der Formel (II):

$$R_F\text{-}R_1\text{-}OH \qquad (II),$$

wobei $R_F$ und $R_1$ wie oben definiert sind, und zwar mit einem Alkylhalogenid mit der Formel (III):

$$R_2\text{-}X \qquad (III),$$

wobei $R_2$ wie oben definiert ist und X ist ein Halogen, gewählt aus Br und I, vorzugsweise Br,
wobei die Reaktion bei einem basischen pH-Wert in einer Lösung erfolgt, die Wasser und zumindest eine wenigstens teilweise wasserlösliche organische Lösung enthält, welche in der Lage ist, wenigstens teilweise den fluorierten Alkohol mit der Formel (II) aufzuschliessen.

**3.** Verfahren nach Patentanspruch 2, ausserdem enthaltend, zusätzlich zu der Reaktionsmischung, wenigstens einen Phasentransferkatalysator.

**4.** Verfahren nach Patentanspruch 2 oder 3, bei welchem die Reaktion bei einer Temperatur von 25°C bis 100°C erfolgt, vorzugsweise von 40°C bis 70°C, und zwar für eine Dauer von generell 2 bis 40 Stunden, vorzugsweise von 4 bis 10 Stunden.

**5.** Mischung aus wenigstens einem Fluoralkyloxalkan nach Patentanspruch 1 und wenigstens einem Silikonöl.

**6.** Mischung nach Patentanspruch 5, enthaltend von 10% bis 95% Gewichtsanteil, vorzugsweise von 20% bis 80% Gewichtsanteil, von wenigstens einem Fluoralkyloxalkan mit der Formel (I), und von 5% bis 90% Gewichtsanteil, vorzugsweise von 20% bis 80%, von wenigstens einem Silikonöl.

**7.** Mischung nach Patentanspruch 5 oder 6, bei welcher das genannte, wenigstens eine Silikonöl eine Viskosität von 100 bis 100.000 cSt (Zentistokes) hat, gemessen bei 20°C.

8. Fluoralkyloxalkan nach Patentanspruch 1 zur Verwendung als Medikament, insbesondere in der Ophthalmologie.

9. Fluoralkyloxalkan nach Patentanspruch 1 zur Verwendung als Tamponadeflüssigkeit bei einer Operation zur Behandlung der Netzhautablösung.

10. Fluoralkyloxalkan nach Patentanspruch 1 zur Verwendung als Glaskörperersatz.

11. Fluoralkyloxalkan nach Patentanspruch 1 zur Verwendung als Agens für oxydierende biologische Gewebe.

12. Fluoralkyloxalkan nach Patentanspruch 1 zur Verwendung als Medikamententräger.

13. Mischung nach einem jeden der Patentansprüche von 5 bis 7 zur Verwendung als Medikament in der Ophthalmologie.

14. Mischung nach einem jeden der Patentansprüche von 5 bis 7 zur Verwendung als Tamponadeflüssigkeit bei einer Operation zur Behandlung der Netzhautablösung.

15. Mischung nach einem jeden der Patentansprüche von 5 bis 7 zur Verwendung als Glaskörperersatz.


**Revendications**

1. Fluoroalkyloxy-alcane ayant la formule :

$$CF_3(CF_2)_5CH_2CH_2O(CH_2)_2CH_3$$

ou

$$CF_3(CF_2)_3CH_2CH_2O(CH_2)_2CH_3.$$

2. Un procédé de préparation d'un fluoroalkyloxy-alcane selon la revendication 1, le fluoroalkyloxy-alcane ayant la formule (I) :

$$R_f\text{-}R_1\text{-}0\text{-}R_2 \qquad (I)$$

dans laquelle :

$R_f$ représente un groupe perfluoroalkyle linéaire, ayant 4 ou 6 atomes de carbone ;
$R_1$ représente un groupe alkylène non fluoré linéaire, ayant 2 atomes de carbone ;
$R_2$ représente un groupe alkyle non fluoré linéaire, ayant 3 atomes de carbone ;

comprenant l'étape consistant à faire réagir un alcool fluoré ayant la formule (II) :

$$R_F\text{-}R_1\text{-}OH \qquad (II)$$

dans laquelle $R_F$ et $R_1$ sont tels que définis ci-dessus, avec un halogénure d'alkyle ayant la formule (III) :

$$R_2\text{-}X \qquad (III)$$

dans laquelle $R_2$ est tel que défini ci-dessus et X est un halogène choisi entre le brome Br et l'iode I, de préférence le brome Br,
où la réaction a lieu à un pH basique dans un solvant comprenant de l'eau et au moins un solvant organique au moins partiellement soluble dans l'eau, qui est à même de solubiliser au moins partiellement l'alcool fluoré de formule (II).

3. Le procédé selon la revendication 2, comprenant en outre l'étape consistant à ajouter au mélange de réaction au moins un catalyseur de transfert de phase.

4. Le procédé selon la revendication 2 ou 3, dans lequel la réaction a lieu à une température de 25°C à 100°C, de préférence de 40°C à 70°C, pendant une période de temps généralement de 2 à 40 heures, de préférence de 4 à 10 heures.

5. Un mélange d'au moins un fluoroalkyloxy-alcane selon la revendication 1 et d'au moins une huile silicone.

6. Le mélange selon la revendication 5, comprenant de 10 % à 95 % en poids, de préférence de 20 % à 80 % en poids, d'au moins un fluoroalkyloxy-alcane de formule (I), et de 5 % à 90 % en poids, de préférence de 20 % à 80 % en poids, d'au moins une huile silicone.

7. Le mélange selon la revendication 5 ou 6, dans lequel ladite au moins une huile silicone a une viscosité comprise entre 100 et 100 000 cS (centistokes), mesurée à 20°C.

8. Un fluoroalkyloxy-alcane selon la revendication 1 destiné à être utilisé en tant qu'un médicament, en particulier en ophtalmologie.

9. Le fluoroalkyloxy-alcane selon la revendication 1 destiné à être utilisé en tant qu'un liquide de tamponnement dans une opération pour le traitement d'un décollement de la rétine.

10. Le fluoroalkyloxy-alcane selon la revendication 1 destiné à être utilisé en tant qu'un substitut de corps vitré.

11. Le fluoroalkyloxy-alcane selon la revendication 1 destiné à être utilisé en tant qu'un agent pour l'oxygénation de tissus biologiques.

12. Le fluoroalkyloxy-alcane selon la revendication 1 destiné à être utilisé en tant qu'un vecteur de médicament.

13. Le mélange selon l'une quelconque des revendications de 5 à 7 destiné à être utilisé en tant qu'un médicament en ophtalmologie.

14. Le mélange selon l'une quelconque des revendications de 5 à 7 destiné à être utilisé en tant qu'un liquide de tamponnement dans une opération pour le traitement d'un décollement de la rétine.

15. Le mélange selon l'une quelconque des revendications de 5 à 7 destiné à être utilisé en tant qu'un substitut de corps vitré.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 0089232 A2
- US 6262126 B1
- WO 2005117850 A
- US 6060626 A
- JP 10175899 A
- EP 0203958 A
- WO 9640834 A
- WO 9739081 A

### Non-patent literature cited in the description

- **C.H. DEPUY et al.** Gas-phase SN2 and E2 reactions of alkyl halides. *J. Am. Chem. Soc.,* 1990, vol. 112, 8650-8655
- **SELVE CALUDE et al.** Synthesis of monodisperse perfluoroalkyl oxyethylene surfactants with methoxy capping: surfactants of high chemical inertness. *Journal of the Chemical Society,* 01 January 1990, 911-912
- **M.B. SMITH ; J. MARCH.** March's advanced Organic Chemistry, Reactions, Mechanisms, and Structure. Wiley-Interscience, 2001
- New fluorous/organic biphasic systems achieved by solvent tuning. **CHU et al.** Tetrahedron. Elsevier Science Publishers, 16 August 2007, vol. 63, 9890-9895
- **BAZHIN D N et al.** synthesis of polyfluorinated Ethers. *Russian Journal of applied Chemistry,* 01 October 2005, vol. 78 (10), 1646-1650